# EUROPEAN PATENT APPLICATION

(11) **EP 0 919 230 A1**
(43) Date of publication of application: **02.06.1999**
(21) Application number: 97203752.7
(22) Date of filing: 01.12.1997
(51) Int. Cl.: A61K 31/20

(54) **NMIFA's as anti-inflammatory agents in superficial mammal tissues**

(71) Applicant: SOCIETE DES PRODUITS NESTLE S.A., 1800 Vevey (CH)
(72) Inventor: Berger, Alvin, 1012 Lausanne (CH)

(57) **Abstract**

A topical pharmaceutical or cosmetic composition comprising, as the active ingredient, at least one of the NMIFA's having the following formula, wherein the NMIFA is under the form of an acid, a salt or an ester, and R1 is C1-C5 alkyl and R2 is C2-C6 alkyl, preferably wherein the NMIFA is 20:3(5,11,14). These NMIFA's are used for the preparation of a composition intended to modulate the metabolism of lipids in superficial mammal tissues.

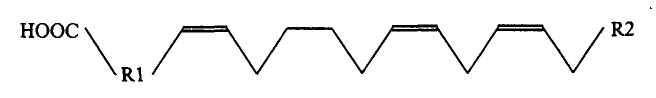

## Description

The present invention relates to the new use of a non-methylene interrupted fatty acid in the prevention and/or the treatment of inflammation in superficial mammalian tissues.

### Background of the invention

The use of fatty acids for preventing and/or treating inflammation in superficial tissues has been described in the literature.

For example, EP5472705 (Prospa B.V.) discloses a new pharmaceutical compositions for topical use containing esters of ω-3 polyunsaturated acids having a high concentration intended for treating psoriasis, phlebitis and correlated pathologies.

EP582239 (Rhone Poulenc Rorer) discloses topical pharmaceutical and cosmetic compositions containing linoleic acid or derivatives as the active ingredient, and a carrier for transporting the active ingredient into the skin. These compositions are used for the prophylaxis and treatment of impure skin, e.g. skin affected by pimples, pustules, urticaria or comedones, of acne and acne-associated skin disorders.

Furthermore, EP5312834 (Woobang Land Co.) discloses a pharmaceutical composition for treating acne including eicosapentaenoic acid and α-linolenic acid, in a weight ratio of 1:0.1 to 20:0.1 of eicosapentaenoic acid to α-linolenic acid respectively. These fatty acids may be extracted from natural substances such as fish oil and/or perilla oil, for example.

Non-methylene-interrupted fatty acid, the acronyme of which is NMIFA, refers to a fatty acid with a series of double bonds in which at least one adjacent pair of double bonds is separated by at least two carbon atoms, i.e., by a group other than a single methylene group. NMIFA's have been the subject of only a few studies in attempts to develop an understand their incorporation into mammal tissues and their potential role in the treatment of certain diseases.

For example, JP61058536 (Nippon Oil) discloses a method for purifying pine nut oil containing at least 10% by weight of 5,9,12-cis-octadecatrienoic acid which exhibits curative effect against high blood pressure.

WO9605164 (Broadben Nominees Pty) discloses an anti-inflammatory preparation comprising a purified active fraction, e.g. 5,11,14,17-eicosatetraenoic acid, isolated from a lipid extract of *Perna canaliculus* or *Mytilus edulis.*

WO9517897 (The Regents of the University of California) shows that broad class of NMIFA's, including 5,11,14-eicosatrienoic acid, may be used in an effective amount for suppressing autoimmune diseases in general, e.g. rheumatoid arthritis, lupus erythmatosis, multiple sclerosis, myasthenia gravis, and about 30 other diseases currently known.

Incorporation of dietary 5,11,14-eicosatrienoate into various mouse phospholipid classes and tissues has been studied. Results show that 5,11,14-eicosatrienoate feeding resulted in lower levels of 20:4n-6 in the hepatic phosphatidylinositol pool. Because leukotrienes and prostaglandins cannot be formed from 5,11,14-eicosatrienoate due to the lack of an internal Δ8 double bond, and because 20:4n-6 was dramatically reduced in some phosphatidylinositol pools, it was expected that dietary intake of 5,11,14-eicosatrienoate may alter eicosanoid metabolism, thus reducing potential inflammation in the hepatic system (Biochemica et Biophysica Acta, 1085, 371-376, 1991; J. Nutr. Biochem., 4, 409-420, 1993).

So far, the following class of NMIFA's, especially 5,11,14-eicosatrienoic acid, has not been reported of being capable to be incorporated into the lipid fraction of superficial mammal tissues. An anti-inflammatory effect in superficial mammal tissues has neither been expected.

### Summary of the invention

Accordingly, it has been found that the NMIFA's having the following formula, wherein the NMIFA is an acid, a salt or an ester, and R1 is C1-C5 alkyl and R2 is C2-C6 alkyl, may be advantageously used for the preparation of a composition intended to modulate the metabolism of lipids in superficial mammal tissues.

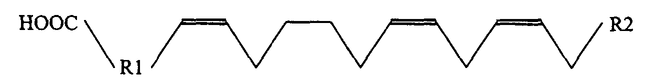

Particularly preferred NMIFA's are those in which R1 is C3 alkyl and R2 is C2-C6 alkyl, or in which R2 is C4 alkyl and R1 is C1-C5 alkyl. The most preferred is that in which R1 is n-propyl and R2 is n-butyl (5,11,14-eicosatrienoic acid, also called 20:3(5,11,14)).

The NMIFA's of the invention may also be used in this context for the preparation of a composition intended to treat or prevent inflammations in superficial mammal tissues by modulating the metabolism of the lipids.

The invention has also for object any topical pharmaceutical and cosmetic compositions comprising the NMIFA's of the invention as the active ingredient.

In a last aspect, the invention provides a pharmaceutical, food or cosmetic composition comprising a combination of fish oil and the NMIFA's of the invention.

The NMIFA's of the invention offer similar advantages as fish oils known to one skilled in the art. However, they provide the advantage of being less oxidizable than fish oil, since they have only 2 methylene interrupted bonds as compared to docosahexaenoic acid in fish oil having 6 methylene interrupted bonds. In addition the NMIFA's of the invention are not a substrate for prostaglandin and leukotriene production, whereas prostaglandin and leukotriene can be formed from the fatty acids found in fish oil, such as 20:5n-3 and 22:6n-3. A further advantage over fish oil preparation lies with the "non-fishy" odour.

### Detailed description of the invention

"Modulation of the metabolism of lipids" is understood as meaning more particularly catabolism of the lipid mediators associated with inflammation, differentiation, proliferation and/or barrier function of superficial tissues.

Furthermore, inflammation of superficial tissues must be understood as the physiological phenomenon involving the production of pro-inflammatory cytokines, such as Tumor Necrosis Factor alpha (TNFα), by the cells of superficial tissues, for example the keratinocytes and the epithelial cells of the cornea, and the cells of the immune system which are contained in these tissues (lymphocytes, Langerhans' cells and the like). The inflammation may result, for example, from an infection, an allergy, a wound and an exposure to radiation and/or irritating agents and/or sensitizing agents.

The fatty acid which is the subject of this invention is a polyunsaturated fatty acid which is linear and monocarboxylic, with all double bonds being cis-double bonds. Several types of nomenclature are used in this specification, and these are as follows.
a) Nomenclature for individual compounds indicating number of carbon atoms and number and position of double bonds, typified by "20:4(5,8,11,14)" for arachidonic acid: the number preceding the colon is the total number of carbon atoms, the number immediately following the colon is the number of double bonds, and the numbers in parentheses are the position of the double bonds, starting from the end of the chain bearing the carboxylic acid group. In all compounds referred to in this manner, except where otherwise indicated, all double bonds are cis.
b) Nomenclature for classes of compounds indicating the location of the double bond closest to the methyl end group, typified by "n-3" or "n-6": the number following the dash denotes the position of the double bond closest to the methyl end of the molecule, counting from the methyl end. Thus, arachidonic acid is in the n-6 class, as is 5,11,14-eicosatrienoic acid (20:3(5,11,14,)), whereas 5,8,11,14,17-eicosapentaenoic acid (20:5(5,8,11,14,17)) is in the n-3 class. This nomenclature is equivalent to "ω" nomenclature in the literature, "ω" and "n" being interchangeable.

Some of the NMIFA's of the invention are naturally occuring subtances. Others may be synthetized according to the well known published methodology (see for example Evans *et al.*, Chem. Phys. Lipids, 38, 327-342, 1995).

For example, 20:3(5,11,14) is a naturally occurring substance which generally occurs as one fatty acid in a mixture of fatty acids. This NMIFA is found in a wide variety of plants as minor or major fraction of the total fatty acid composition. Both the extraction of the fatty acid mixtures from their natural sources and the extraction of the 20:3(5,11,14) from the remaining fatty acids can be achieved by conventional extraction and purification procedures well known among those skilled in the art.

The natural sources of fatty acids containing 20:3(5,11,14) are primarily plant seeds, and prominent among these are conifers and ornamental shrubs. The seed oils from these plants are similar to normal edible oils, containing largely oleic, linoleic and linolenic acids, but also containing useful amounts of NMIFA's. Table 1 lists examples of seeds whose lipid contents contain significant amounts of 20:3(5,11,14).

**Table 1**

| Source | % of 20:3(5,11,14) among total fatty acids | Source | % of 20:3(5,11,14) among total fatty acids |
|---|---|---|---|
| *Juniper virginiensis* | 14.8 | *Sciadopitys verticallata* | 15 |
| *Plarycladus orientalis* | 3 | *Caltha palustris* | 23 |
| *Juniperis chinesis* | 12.3 | *Calitrus rhomboidea* | 14 |
| *Torreya nucifera* | 7 | *Mortierella alpina**** | 7 |
| *Podocarpus nagi* | 24 | *Ephedra campylopoda* | 22 |
| *Anemone rivularis* | 10 | *Anenome leveillei* | 6 |
| *Cimcifuga racemosa* | 6 | *Erantis hyemalis* | 6 |
| *Gingko biloba* | 2.2 | *Pinus silvestris* | 7 |

| | | | |
|---|---|---|---|
| * see the Japanese patent JP5276964 (Suntory LTD) | | | |

Purification of 20:3(5,11,14) may be in particular achieved by (1) choosing a starting seed source high in total fat content and 20:3(5,11,14) content but not containing other contaminating trienes, in particular alpha-linolenic acid (18:3n-3) and gamma-linolenic acid (18:3n-6) (Podocarpus nagi, Table 1, is such an example); (2) extracting the lipids with isopropanol and chloroform according to the method of Nichols (Biochim. Biophys Acta 70: 417, 1963); (3) conventional degumming and decoloring procedures; (4) preparing methyl esters with 2% methanolic sulfuric acid according to the method of Christie (p. 52-53, in Lipid Analysis, Pergamon Press, Oxford, 1982); (3) eluting 20:3(5,11,14) methyl ester from a silver nitrate impregnated acid-washed florisil column with a hexane:ether mixture ranging from 9:1 to 8:2 (volume/volume) (after Carroll, J. Am. Oil Chem. Soc. 40: 413, 1963; Wilner, Chem. Ind (Lond) Oct 30: 1839, 1965; Merck ChromNews 4(1): 1995; Anderson, J. Lipid Res. 6: 577, 1965; and Teshima, Bull. Jap. Soc. Scien. Fish. 44: 927, 1978); (4) removing contaminating silver ions by the method of Åkesson (Eur. J. Biochem. 9: 463, 1969); and (5) optionally converted the methyl ester back to the free acid form by saponification in 1 M potassium hydroxide in 95% ethanol after Christie (p. 51-52, in Lipid Analysis, Pergamon Press, Oxford, 1982).

In the context of the present invention, the NMIFA's of the invention may be used as an acid, a salt or an ester, for example as a methyl ester, ethyl ester, mono-, di-, tri-glyceride or phospholipid ester. The NMIFA's of the invention may be used in a purified form, or also as a mixture of fatty acids present in an oil extracted from one of the plants described above, for example.

Administration of the NMIFA's may be achieved by known methods known to one skilled in the art, to any kind of superficial mammal tissues, that is to say to cells which make up the skin, the scalp, the eye, or the oral, buccal, nasal and vaginal mucosa, for example.

The NMIFA's may used for the treatment or prophylaxis of diseases of the skin or of the scalp, in particular against inflammations associated with, for example, psoriasis, erythema (sunburn), eczema, seborrhoeic dermatitis, alopecia areata, mycose, acne and other dermatoses.

A composition of the invention, intended for topical applications, may thus comprise an effective amount of the NMIFA's as the active ingredient, and a carrier for transporting the active ingredient into superficial mammal tissues.

The carrier may be any kind of carriers known to one skilled in the art for cosmetic or pharmaceutical formulations. Liposomes may thus be one of the carrier used in that context. These molecules include one or more various types of substances including nonpolar lipids, polar lipids, mono- and diglycerides, sulfatides, lysolecithin, phospholipids, saponin, bile acids and salts. Liposomes can exist as emulsions and foams, micelies, insoluble monolayers, liquid crystals, phospholipid dispersions or lameliar layers. The NMIFA's can be incorporated in the liposome, optionally in conjunction with an appropriate ligand or mimetic which binds to specific cell receptors.

Liposomes are generally formed from standard vesicle forming lipids, which generally include neutral and negatively charged phospholipids and a sterol, such as cholesterol. The selection of lipids is generally guided by consideration of various factors, such as for example the desired liposome size and the need for stability of the liposomes in the bloodstream. Typically, the major lipid component in the liposomes is phosphatidylcholine. Partially hydrogenated egg phosphatidylcholine is a typical example.

Liposomes typically contain about 5-15 mole percent negatively charged phospholipids, such as phosphatidylglycerol, phosphatidylserine or phosphatidylinositol. Negatively charge phospholipids help prevent spontaneous aggregation of the liposomes and thus lower the incidence of aggregates formed from undersized liposomes. Membrane rigidifying agents, such as sphingomyelin or a saturated neutral phospholipid, at a concentration of at least about 50 mole percent, and 5-15 mole percent of monosialylganglioside, may also be included to provide increased circulation of the liposome preparation in the bloodstream.

Liposome suspensions may also include lipid-protective agents to protect the lipids from free-radical and lipid-peroxidative damage during storage. Examples of such agent are lipophilic free-radical quenchers such as alpha tocopherol, and water-soluble ironspecific chelators such as ferrioxiamine.

Liposomes can be prepared by a variety of methods known among those skilled in the art. The liposomes can then be sized to a desired size range and a relatively narrow size distribution. A size range which permits the liposome suspension to be sterilized by filtration through a conventional filter, for examples, is about 0.2-0.4 microns.

Formulations for administration will generally comprise a solution of the compound dissolved or suspended in an acceptable vehicle, the appropriate choices of which will readily occur to those skilled in the art. The formulations may further contain pharmaceutically acceptable auxiliary substances as required to approximate physiological conditions, such as pH adjusting and buffering agents, tonicity adjusting agents, and wetting agents.

The cosmetic or pharmaceutical composition may be applied directly to the superficial tissues such that the NMIFA's diffuse through the cells and be assimilated there. This composition can also be injected underneath the superficial tissues, for example by means of a subcutaneous injection. In both cases, the application is regarded as topical, that is to say it is applied directly onto or underneath the superficial tissues.

Application of the NMIFA's can also be extended to inflammations of the eye, more particularly of the cornea, and also of the mucosa, more particularly of the oral, nasal, buccal, anal and vaginal mucosa. Compositions administered orally may affect directly the buccal, oesophageal, gastric and intestinal mucosa, and/or may pass into the bloodstream and be carried directly, for example, to the cells of the skin, the eye or the mucosa.

This composition can also be applied into the nasal passages by means of a diffuser, a gel and/or a physiological liquid for conventional flushing of the nasal passages.

It should be pointed out that, depending on the galenical forms usually used for topical or oral application, that is to say depending on the dietary, cosmetic and/or pharmaceutical forms, the amount of the NMIFA's sufficient and necessary to observe an anti-inflammatory effect or an effect as a modulator of the metabolism of lipids may vary considerably. The invention thus relates to the use of the NMIFA's in an amount sufficient for treatment or prevention of inflammations and/or for modulations of the metabolism of lipids of the superficial tissues.

The NMIFA's thus be administered to human or animal, for example, as a food material to treat oral mucosa. It may thus be used as replacement for the oils normally used in food formulations or recipes, or as part of a mixture of oils used in this manner. This composition can be, for example, a sauce, such as a salad sauce, a table oil, a mayonnaise, an ice cream, a confectionery composition or a filling paste or spreading paste. The compound can also be administered as part of nutritional supplement, such as tablets or capsules taken orally on a dailly basis. Binders, matrices, and other conventional adjuvants normally found in supplements of these types will generally be included here as well. Typical dosages for such methods may vary widely, but will most often fall within the range of about 2 mg per kg of body weight to about 2000 mg per kg, and more often within the range of about 5 to about 500 mg per kg.

The food composition may preferably comprise a carrier to transport the NMIFA's to the large bowel or other region of the gastrointestinal tract. The carrier may be a resistant starch incorporated at a level of 2 to 20% by weight. Incorporation can be achieved while drying together the food components, by freeze drying, for example. Incorporation can also be achieved by microencapsulation. A typical process for microencapsulation requires the preheating of a low melting point oil above its melting point (<40°C), the NMIFA's are then added to the mixture, resistant starch is also added together with other ingredients and binding agents such as gelatin and gums, and the dispersion is sprayed into the head of a coolong tower to allow uniform particles to form with an average size typically in the range 20-200 microns. A preferred form of resistant starch is a high amylose starch, for example those disclosed is WO94/03049 and WO94/14342.

In the field of human or animal cosmetics, the invention also relates to the use of the NMIFA's for the preparation of a galenical form usually used for topical or oral hygiene application, and in particular in the form of oily, alcoholic or aqueous-alcoholic solutions, gels, water-in-oil or oil-in-water emulsions having the appearance of a cream or a gel, if necessary capable of foaming, in the form of an aerosol, or also in the form of vesicular dispersions comprising ionic and/or non-ionic lipids. These galenical forms are in all cases prepared by the usual methods of the cosmetic fields under consideration. This composition can be, for example, in particular a composition for cleansing, protection, treatment or care for the scalp, for the face, for the neck, for the hands, or for the body (for example in the form of vanishing creams, night creams, make-up remover creams, suncreams or oils, oils for the body or the face, cleansing milks, make-up remover milks or body milks), a make-up composition (fore example make-up foundation), an artificial tanning composition, a composition for bathing, a shampoo composition (treatment of the scalp) or a composition for buccal hygiene (mouthwash, toothpaste or chewing gum).

In the field of human or animal pharmacy, in particular dermatology, oto-rhinolaryngology, ophthalmology, gastroenterology and gynaecology, this composition can be, for example, a capsule, a soft gelatin capsule, an emulsion, a pomade, a composition which can be injected underneath the skin, an ointment, a syrup, a diffuser, an eyewash, a shampoo or a mouthwash comprising the NMIFA's for treatment or prophylaxis of the skin, the eye or the mucosa. The various possible galenical forms are in all cases prepared by the usual methods of the pharmaceutical field under consideration.

The present invention is described in more detail by the examples given below. It goes without saying, however, that these examples are given by way of illustration of the subject matter of the invention, of which they constitute no limitation in any manner. The percentages are given by weight, unless indicated otherwise.

### Example 1 Metabolism of 20:3(5,11,14)

Determination of the profile of 5,11,14 in the human immortalized keratinocyte line DK2-NR (described in the patent PCT/EP96/05812) was achieved as follows.

Confluent cultures of DK2-NR were incubated 4 days in NR-2 medium (Biofluids Inc. Rockville, MD, USA) with an increased Ca²⁺-concentration (CaCl₂: 1.5mM). Then, the keratinocyte were incubated 2 times 2 days (medium change with fatty acids after 2 days) in NR-2 containing 1mg/ml bovine serum albumin (BSA, fatty acid free, Sigma Inc. St. Louis, USA) and 15µM of 5,11,14-eicosatrienoic acid methyl ester (5,11,14-ester) and/or arachidonic acid methyl ester (20:4n-6-ester).

Furthermore, the cells were preincubated for two days with 15µM 20:4n-6-ester (see table N° 6-9) and subsequently for 2x2 days with 5,11,14-ester and with a mixture of 20:4n-6-ester and 5,11,14-ester.

At the end of the incubation time the cells were washed in HBSS (Hanks Balanced Salt Solution (Gibco, Life Technologies) containing 0.1% BSA. And two times in HBSS. The cells were harvested by scraping (cell scraper, Costar Inc.) the cells from the culture dishes. The cells were centrifuged in 1 ml HBSS (1000 rpm). The cell extraction was carried out in a mixture of hexan/isopropanol (2:1 v/v) containing 2,6-di-*tert*-butyl-*p*-cresol. The phospholipids were separated by chromatography (silica gel 60) with the eluent chloroform/acetone (96:4 v/v). The fatty acid fraction of the phospholipids were then esterified by heating (100°C) in a solution containing 10% BF3-methanol (Supelco, Bellefonte, PA, USA). The metyl esters were separated. The fatty acids were quantified according to internal standards.

The results listed in table 2 below show that 5, 11, 14-eicosatrienoic acid was integrated into the total lipids (3.34%).

**Table 2**

| | Treatment with fatty acids | | | Fatty acids incorporated phospholipids in cellular | | | |
|---|---|---|---|---|---|---|---|
| N° | Day 0 | Day 2 | Day 4 | 5,11,14 % | 20:3n-6 % | 20:4n-6 % | 20:5n-3 % |
| 1 | - | Ethanol | Ethanol | 0.00 | 0.11 | 0.43 | 0.04 |
| 2 | - | 5,11,14-ester | 5,11,14-ester | **3.34** | 0.17 | 0.47 | 0.06 |
| 3 | - | 20:4n-6-ester | 20:4n-6-ester | 0.30 | 0.28 | 8.25 | 0.04 |
| 4 | - | 5,11,14-ester 20:4n-6-ester | 5,11,14-ester 20;4n-6-ester | 1.04 | 0.58 | 9.56 | 0.00 |
| 5 | - | 20:4n-6-ester | 20:4n-6-ester | 0.30 | 0.55 | 10.39 | 0.00 |

| | Pre-incubation | | | | | | |
|---|---|---|---|---|---|---|---|
| 6 | 20:4n-6-ester | 5,11,14-ester | 5,11,14-ester | **2.12** | 0.38 | 5.30 | 0.03 |
| 7 | 20:4n-6-ester | Ethanol | Ethanol | 0.00 | 0.30 | 5.57 | 0.01 |
| 8 | 20:4n-6-ester | 20:4n-6-ester 5,11,14-ester | 20:4n-6-ester 5,11,14-ester | 0.98 | 1.05 | 12.90 | 0.01 |
| 9 | 20:4n-6-ester | 20:4n-6-ester + Ethanol | 20:4n-6-ester + Ethanol | 0.09 | 0.85 | 11.80 | 0.03 |

### Example 2 Effect of 5,11,14-eicosatrienoic acid on the TNFα-secretion of Ultraviolet B treated human immortalized keratinocytes

Immortalized human keratinocytes (DK2-NR) were incubated in culture plates (diameter: 3.5cm) with 1.5ml NR-2 medium. After reaching confluency the Ca²⁺-concentration in NR-2 medium was shifted to 1.5mM. After 4 days, the cells were incubated 2x2 days in NR-2 without hydrocortisone, with high Ca²⁺ (1.5mM) and various fatty acids (i.e. 18:2n-6, 18:3n-3, 20:3(5,11,14)). At the end of the incubation period the NR-2 medium was removed and stored at 37°C to re-feed the cells after the UVB-treatment (conditioned NR-2 medium). The cell cultures were washed 2x with HBSS. For the UVB-treatment the cells were incubated in 0.5 ml HBSS. The cell cultures were treated with 1.5kJ UVB (Phillips, TL100, maximal emission at 313 nm). Then, HBSS was replaced with the corresponding conditioned NR-2 medium. The supernatant was collected after 24hours and was stored at -20°C. The secreted TNFα concentration in the supernatant was quantified by ELISA (Pelikine Compact, CLB Amsterdam, The Netherlands).

As listed in table 3 it could be shown that 5,11,14-eicosatrienoic acid inhibits the secretion of TNFα (9pg/ml) in UVB-treated human immortalized keratinocytes compared to controls (11.6pg/ml).

**Table 3**

| | Secreted TNFα [pg/ml] | ± SD |
|---|---|---|
| No UVB + control (EtOH) | 0.0 | 0 |
| **UVB + control (EtOH)** | **11.6** | **0.009** |
| **UVB + 5,11,14** | **9.0** | **0.005** |
| UVB + 20:4n-6 (arachidonic acid) | 5.1 | 0.016 |
| UVB + 18;3n-3 (α-linolenic acid) | 14.5 | 0.015 |
| UVB + 18:1n-9 (oleic acid) | 12.1 | 0005 |
| UVB + Hydrocortisone (0.5µg/ml) | 2.0 | 0.004 |

### Example 3 Effect of 5,11,14-eicosatrienoic acid on the TNFα-secretion of TPA-treated human immortalized epithelial cell line

Human epithelial colon cells express naturally proteins and enzymes involved in inflammatory responses after treatment with pro-inflammatory agents, like phorbol esters. Among proteins and enzymes thus produced, one may discriminate the superoxide-dismutase (SOD) and the tumoral necrosis factor (TNFα), for example.

In the following assays, human epithelial cells of the colon in culture, primary or immortalised, are *in-vitro* subjected to an inflammatory treatment with 10ng/ml of 12-*O*-tetradecanoylphorbol acetate (TPA). The immortalised cell line described in EP96201064.1 (Société des Produits Nestlé) was used in this context. This cell line has been deposited, according to the Budapest Treaty, at the Deutsche Sammlung von Mikroorganismen und Zellkulturen, Mascheroder Weg 1b, D-38124 Braunschweig, Germany, in Avril 16^{th} , 1996, where it receives the deposit number DSM ACC2258.

Accordingly, Petri dishes (6 cm diameter) containing 4 ml of the culture medium B50 (described in EP96201064.1) are seeded with primary or immortalised above-mentioned lines. At confluence, the medium is replaced with fresh medium B50 rich in calcium (CaCl2, 1,5 mM). Incubation occurs during 2 days, the medium is against replaced with fresh medium, and incubation occurs again during 2 days. Cells are fed then during 2 days with B50 medium rich in calcium (CaCl2, 1,5 mM), containing 1 mg/ml of BSA and a given fatty acid (oleic acide or 5,11,14 eicosatrienoic acide) or an equivalent amount of solvent (ethanol).

During the last 24h of incubation, 10ng/ml of TPA or an equivalent amount of ethanol is added to the medium. At the end, supernatants are collected and the content of TNFα thus produced is measured by ELISA (specific TNFα antibodies are available: BioSource Inter., US, n° KHC0012 et KHC3013).

Results show that incubation of human colon cells with 5,11,14-eicosatrienoic acid leads to an inhibition of TNFα production by. This inhibition is specific since oleic acid (the control) has no inhibitory effects.

### Example 4 Effect of 5,11,14-eicosatrienoic acid on the TNFα-secretion of TPA-treated human immortalized corneal cell line

Human comeal epithelial cells naturally express enzymes involved in inflammatory responses after treatment with pro-inflammatory agents, like phorbol esters. Among proteins and enzymes thus produced, one may discriminate the collagenase I (Bazan *et al*., Proc. Natl. Acad. Sci. USA, 90, 8678-8682, 1993), c-Fos (see also Bazan *et al.*) and the arachidonic intermediates 12-hydroxyeicosatetraenoic acid and 12-hydroxyeicosatrienoic acid (Conners *et al*., Inves. Ophth. & Visu. Sci., 36, 828-840, 1995), for example.

In the following assays, human epithelial cells of the cornea in culture, primary or immortalised, are *in-vitro* subjected to an inflammatory treatment with 10ng/ml of 12-*O*-tetradecanoylphorbol acetate (TPA). The immortalised cell line described in EP96203707.3 (Société des Produits Nestlé) was used in this context. This cell line has been deposited, according to the Budapest Treaty, at the Collection Nationale de Culture de Microoganismes (CNCM), 25 rue du Docteur Roux, 75724 Paris, in October 22^{nd}, 1996, where it received the deposit number CNCM I-1777. Culture medium used is NR-2.

As described in example 3, the effect of 5,11,14-eicosatrienoic acid on the collagenase I production from corneal cells is determined after irritating treatment. To this end, expression of collagenase I is detected by Polymerase Chain Reaction (PCR), Western-Blot or ELISA.

For detecting collagenase I by PCR, RNA is extracted from sensitised cells, and collagenase I mRNA is detected by PCR with a primer derived from the collagenase I DNA sequence (Genebank: n°X05231).

For detecting collagenase I by Western-Blot, the sensitised culture medium is recuperated, concentrated by use of Amicon30® filter. Proteins are separated on SDS-PAGE polyacrylamide gel, transferred by Western-blot on a filter, put in contact with a first anti-collagenase I antibody (Cortex Biochem, US, n° 60338P), to a second antibody coupled to peroxydase (Pierce, US, n° 31400), then to the peroxydase's substrate.

For detecting collagenase I by par ELISA kit hMMP-1 by Amersham may be used (catalog, rpn 2610), by following indications from the supplier.

Results of the trials are comparable to those obtained from the trials of example 3.

### Example 5 Anti-inflammation effect of other NMIFA's

Determination of the anti-inflammation effect of 18:3(5,11,14), 18:3(3,9,12), 19:3(5,11,14), 19:3(4,10,13), 21:3(5,11,14), 21:3(6,12,15), 22:3(5,11,14), and 22:3(7,13,16) in the human immortalized keratinocyte line DK2-NR (described in the patent PCT/EP96/05812) was achieved as described in example 2 above. Results show that anti-inflammation effects are exhibited.

### Example 6 Body milk (oil-in-water emulsion)

| *Oil phase* : | |
|---|---|
| Glyceryl stearate/PEG-100 stearate (Arlacel 165 Sold by ICI) (emulsifier) | 1 % |
| Polysorbate 60 (emulsifier) | 0.8 % |
| Hydrogenated Polyisobuten | 2 % |
| Stearic acid | 1 % |
| Oil from seed of *Ephedra campylopoda* | 8 % |

| *Water phase* : | |
|---|---|
| Glycerin | 3 % |
| Carbomer (carbopol 941 sold by Goodrich) (thickener) | 0.3 % |
| Triethanolamin (neutralising agent) | 0.3 % |
| Conservative | 0.3 % |
| Water | up to 100% |

The emulsion is prepared by incorporating the oil phase in the water phase while stirring. The body milk provides a good protection of the skin against inflammations.

### Example 7 Care fluid (oil-in-water emulsion)

| *Oil Phase:* | |
|---|---|
| Methylglucose sesquistearate (emulsifier) | 2 % |
| Cyclomethicon | 13 % |
| Oil from seed of *Podocarpus nagi* | 2 % |
| Perfume | 0.2 % |
| PEG 20 methylglucose sesquistearate (emulsifier) | 2 % |

| *Water phase*: | |
|---|---|
| Xanthan gum (thickener) | 0.2 % |
| Polysacrylamide/isoparaffin C13-C14/leureth-7 (Sepigel 305 sold by Seppic) (thickener) | 0.8 % |
| Conservative | 0.3% |
| Water | up to 100% |

Emulsion is prepared as described in example 6. A white fluid is obtained which provides a good protection of the skin against inflammations.

### Example 8 Care cream (water-in-oil emulsion)

| *Oil phase* (A) | |
|---|---|
| Polyglyceryl-4 Isostearate/Cetyl dimethicon copolyol/Hexyl laurate (Abil WE 09 sold by Goldschmidt) (emulsifier) | 4 % |
| Isohexadecan | 5 % |
| Oil from seed of *Caltha palustris* | 10 % |
| Cyclomethicon | 3.5 % |
| n-octanoyl-5-salicylic acid | 1 % |
| Perfume | 0.15 |

| *Water phase* (B) | |
|---|---|
| Glycerin | 10 % |
| Cellulose gum | 0,5 % |
| Magnesium sulphate | 0.65 % |
| Conservative | 0.3 % |
| Water | up to 100 % |

For preparing the emulsion, constituents of phase A are heated at 80°C up to complete dissolving, and are refrigerated at 65°C. Phase B is heated at 65°C, phase A is poured into while stirring, then the mixture is refrigerated. A white fluid is obtained which provides a good protection of the skin against inflammations.

## Claims

1. A topical pharmaceutical or cosmetic composition comprising, as the active ingredient, at least one of the NMIFA's having the following formula, wherein the NMIFA is under the form of an acid, a salt or an ester, and R1 is C1-C5 alkyl and R2 is C2-C6 alkyl:

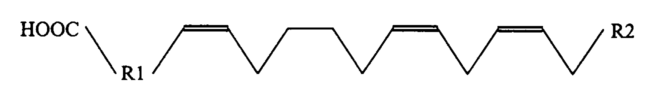

2. A topical pharmaceutical or cosmetic composition according to claim 1, wherein R1 is C3 alkyl and R2 is C2-C6 alkyl, or in which R2 is C4 alkyl and R1 is C1-C5 alkyl.

3. A topical pharmaceutical or cosmetic composition according to claim 2, wherein the NMIFA is 20:3(5, 11, 14).

4. A topical composition according to claim 1 wherein the NMIFA has been previously purified, synthetized, or is present in a mixture of fatty acids.

5. A topical composition according to one of the preceeding claims, comprising an effective amount of a NMIFA as the active ingredient, and a carrier for transporting the active ingredient into superficial mammal tissues.

6. A pharmaceutical, food or cosmetic composition comprising a combination of fish oil and at least one of the NMIFA's defined in claim 1.

7. The use of at least one of the NMIFA's defined in claim 1 for the preparation of a composition intended to modulate the metabolism of lipids in superficial mammal tissues.

8. The use as claimed in claim 7, for the preparation of a composition intended to treat or prevent inflammations in superficial mammal tissues.

9. The use as claimed in claim 7 or 8, wherein the NMIFA is 20:3(5,11,14).
